# EUROPEAN PATENT APPLICATION

(11) **EP 4 544 991 A1**
(43) Date of publication of application: **30.04.2025**
(21) Application number: 24208372.3
(22) Date of filing: 23.10.2024
(51) Int. Cl.: A61B 5/00, A61C 19/04, G06T 7/00

(54) **CONTACTLESS DETECTION OF DEFECTIVE TOOTH MATERIAL**

(30) Priority: 24.10.2023 US 202318493135
(71) Applicant: Dentsply Sirona Inc., York, PA 17401 (US)
(72) Inventor: ERTL, Thomas, 64625 Bensheim (DE); Voss, Bjoern, 64625 Bensheim (DE); FRANKE, Frederike, 64625 Bensheim (DE)
(74) Representative: Venner Shipley LLP

(57) **Abstract**

Techniques and apparatuses for detecting defects on at least one tooth in the dentin or tooth enamel area that include providing an intra-oral camera that includes a confined light injector; projecting a spatially confined light in a first direction via the confined light injector at an illumination point of the at least one tooth; recording, by an image sensor, one or more surface light distribution images received via captured light that is backscattered from the tooth; and computing, by a tooth defect detection module, a defect condition of the at least one tooth by classifying the one or more surface light distribution images based on computation of light distribution differences in the one or more surface light distribution images caused by defective tooth material.

## Description

### BACKGROUND

### Technical Field

The present disclosure generally relates to detecting defective tooth material and more particularly to contactless detection of defective tooth using an intraoral camera configured to confine illumination light to at least one illumination spot and derive surface and subsurface information from the vicinity of the at least one illumination spot.

### Description of the Related Art

Dental radiography and tactile inspection are some of the most commonly used methods for identifying dental caries and other defects, but both have substantial drawbacks. A device may be used in tactile examinations, which may hasten the onset of incurable caries by traumatizing the tooth's structure. Radiography options may use x-ray radiation, an ionizing radiation that may be harmful to the patient's health.

### BRIEF SUMMARY

According to an embodiment of the present disclosure, a method for contactless detection of defective tooth material is disclosed. The method includes providing an intra-oral camera that includes a confined light injector; projecting spatially confined light in a first direction via the confined light injector, at one or more illumination point(s) of the at least one tooth; recording, by an image sensor, one or more light distribution images received via captured light backscattered from the tooth; computing, by a tooth defect detection module, a defect condition of the at least one tooth by classifying the one or more surface light distribution images based on computation of a homogeneity disturbance in the one or more surface light distribution images caused by a defective tooth material. The spatially confined light is projected at the illumination point(s) in a contactless manner.

In an embodiment, a comparator is used to compare the one or more surface light distribution images with a database of stored surface light distribution images that comprise light distribution data of various tooth groups as anteriors, premolars, molars comprising various tooth defects as caries, and demineralization on various tooth surfaces. The database may be obtained based on Monte-Carlo simulations of light propagation.

In an embodiment, a machine-learned model is used to classify the one or more surface light distribution images. The machine-learned model is trained based on at least a plurality of test surface light distribution images that comprise healthy and defective tooth material data.

According to an embodiment, a system is disclosed including an intra-oral camera including a confined light injector, a processor, and a memory storing instructions that, when executed by the processor, configure the system to perform any of the methods described herein.

According to an embodiment, a non-transitory computer readable storage medium is disclosed to store one or more programs that when executed by a processor cause the intra-oral camera to perform any of the methods described herein.

### BRIEF DESCRIPTION OF THE DRAWINGS

To easily identify the discussion of any particular element or act, the most significant digit or digits in a reference number refer to the figure number in which that element is first introduced.
FIG. 1 depicts a block diagram of a network of data processing systems in accordance with an illustrative embodiment.
FIG. 2 depicts a block diagram of a data processing system in accordance with an illustrative embodiment.
FIG. 3 depicts a sketch of a contactless detection of defective tooth material in accordance with an illustrative embodiment.
FIG. 4 depicts a sketch of a contactless detection of defective tooth material in accordance with an illustrative embodiment.
FIG. 5 depicts a lock diagram of an application in accordance with an illustrative embodiment.
FIG. 6 illustrates a high-level block diagram showing a structure of a neural network according to one embodiment.
FIG. 7 illustrates a block diagram illustrating of a training system in accordance with an illustrative embodiment.
FIG. 8 depicts a routine in accordance with an illustrative embodiment.

### DETAILED DESCRIPTION

The illustrative embodiments recognize that to examine the tooth structure with camera/scanner systems using transillumination techniques, a large amount of light of relevant wavelength may be coupled into the tooth to be able to measure a transillumination signal with sufficient signal strength from all areas of the tooth. Further, light may be coupled into the tooth perpendicularly to the observation beam path by physically contacting the tooth with a light source. The illustrative embodiments recognize that this may require sophisticated measuring devices that may necessitate the teeth to be touched by a camera device during image acquisition, thus posing hygiene concerns. The illustrative embodiments recognize that this may further make it difficult, if not impossible to scan an entire jaw without using separate scanners or at least a different scanner head.

The illustrative embodiments disclose confined light injection (such as a laser focused illumination in the visible or near-infrared (NIR) wavelength range) on tooth material such as enamel, dentin, and semitranslucent tooth filling materials to generate a homogeneous background illumination by internal light scattering in the tooth or filling material volume while alleviating or eliminating back reflections. Focusing light on enamel, dentin and semitranslucent tooth restoration material causes the material to scatter the injected light multiple times and illuminates the tooth from inside resulting in a relatively homogeneous background illumination. When tooth defects such as caries, demineralization and tooth cracks are present, the homogenous background illumination is modified by the inhomogeneities in absorption, scattering and refraction index caused by such tooth defects and results in a modified light distribution on the surface of the tooth, which is captured by the camera. The tooth defect modifies the homogenous background illumination by higher light absorption in dentin and higher light scattering in demineralized enamel and a discontinuity of the refraction index in case of a tooth crack.

Due to higher order volume scattering in enamel and dentin, this confined light injection causes a diffuse illumination in part from inside of the entire tooth with a highest intensity at the light injection point(s) and decreasing intensity into the periphery. This illumination from inside allows a diffuse illumination by light injection from the side and/or rear of a defect (e.g., caries) and creates a light distribution on the tooth surface influenced by the defect to be observed. The tooth is then imaged with an image sensor that is sensitive to the corresponding wavelength. In a similar way as the presence of caries alters the background illumination, cracks in enamel become visible in the background illumination due to the formation of a shadow in tooth areas behind the cracks as seen from the illumination points. This can be used to detect cracks in the enamel at least above the gingiva.

Responsive to strong back reflection occurring at an illumination point being masked out, an evaluable transillumination volume scattering image may be obtained in the remaining areas of the image, the content of which can be used to draw conclusions about structural damage to the tooth structure. The illumination point may be computed from the knowledge of the position of the intra-oral camera in relation to the tooth or can be determined by selecting pixels with high exposure values to define the area to be masked around it.

In one aspect, a method for detecting defects on at least one tooth in the dentin or tooth enamel area includes providing an intra-oral camera comprising a confined light injector; projecting a spatially confined light in a first direction or a plurality of directions via the confined light injector, at an illumination point(s) of the at least one tooth so that the confined light is focused on an area of interest such as a portion of the dentin; recording, by an image sensor, one or more surface light distribution images received from captured light backscattered from the tooth; computing, by a tooth defect detection module, a defect condition of the at least one tooth by classifying the one or more surface light distribution images based computation of a homogeneity disturbance in the one or more surface light distribution images caused by defective tooth material.

The illustrative embodiments are described with respect to certain types of data, functions, algorithms, equations, model configurations, locations of embodiments, additional data, devices, data processing systems, environments, components, and applications only as examples. Any specific manifestations of these and other similar artifacts are not intended to be limiting to the invention. Any suitable manifestation of these and other similar artifacts can be selected within the scope of the illustrative embodiments.

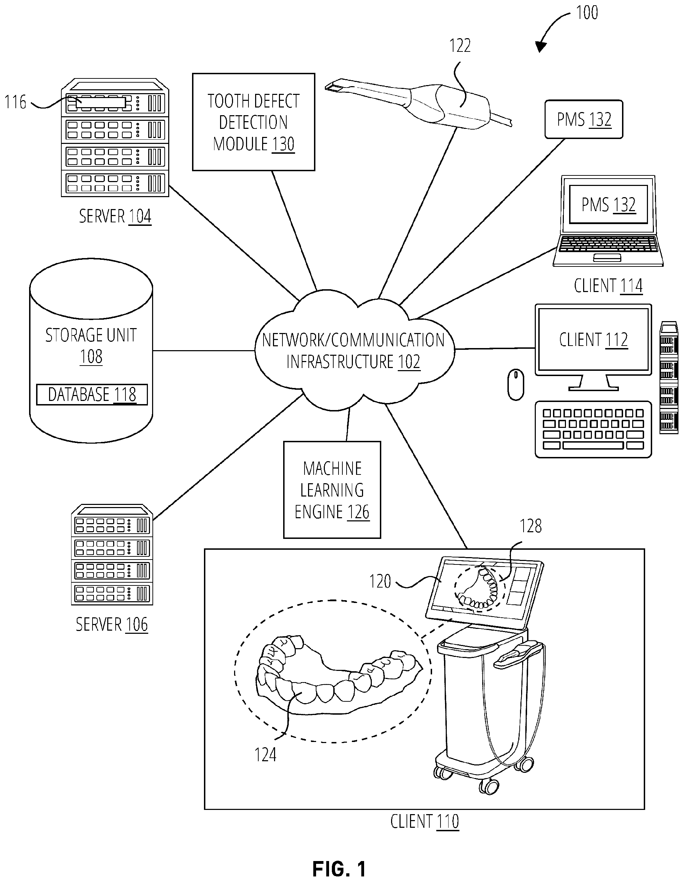

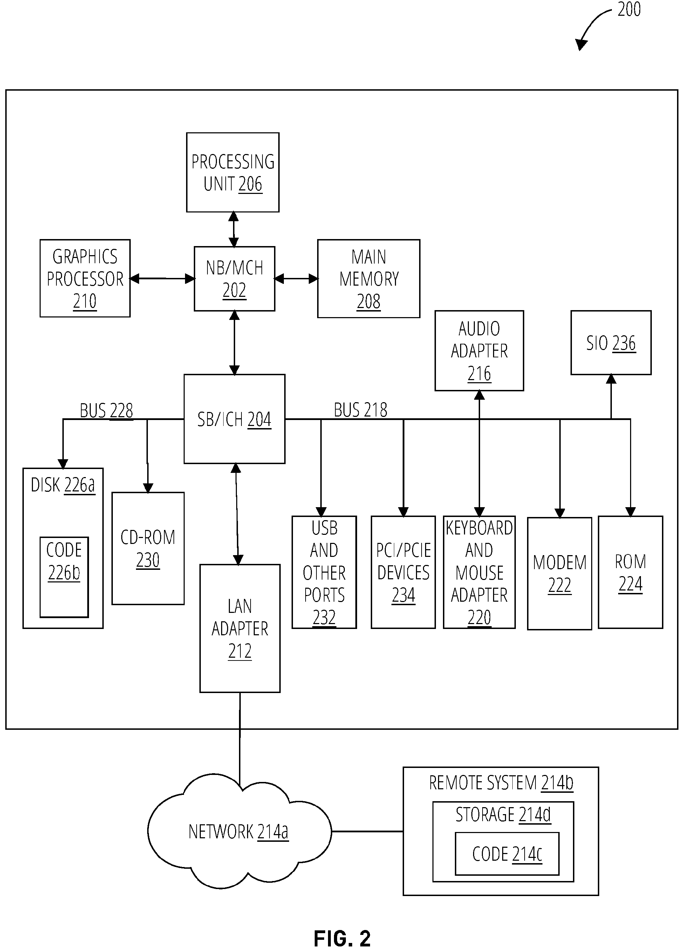

Any advantages listed herein are only examples and are not intended to be limiting to the illustrative embodiments. Additional or different advantages may be realized by specific illustrative embodiments. Furthermore, a particular illustrative embodiment may have some, all, or none of the advantages listed above. With reference to the figures and in particular with reference to FIG. 1 and FIG. 2, these figures are example diagrams of data processing environments in which illustrative embodiments may be implemented. FIG. 1 and FIG. 2 are only examples and are not intended to assert or imply any limitation with regard to the environments in which different embodiments may be implemented. A particular implementation may make many modifications to the depicted environments based on the following description.

FIG. 1 depicts a block diagram of an environment of data processing systems in which illustrative embodiments may be implemented. Data processing environment 100 is a network of computers in which the illustrative embodiments may be implemented. Data processing environment 100 includes network/communication infrastructure 102. Network/communication infrastructure 102 is the medium used to provide communications links between various devices, databases and computers connected together within data processing environment 100. Network/communication infrastructure 102 may include connections, such as wire, wireless communication links, or fiber optic cables.

Clients or servers are only example roles of certain data processing systems connected to network/communication infrastructure 102 and are not intended to exclude other configurations or roles for these data processing systems. Server 104 and server 106 couple to network/communication infrastructure 102 along with storage unit 108. Software applications may execute on any computer in data processing environment 100. Client 110, client 112, client 114 are also coupled to network/communication infrastructure 102. Client 110 may be a dental acquisition unit with a display. A data processing system, such as server 104 or server 106, or clients (client 110, client 112, client 114) may include data and may have software applications or software tools executing thereon. Client 114 or any other clients or data processing systems may include a practice management system 132 (PMS). The PMS 132 may alternatively, or in addition be a standalone system use in a practice for managing patient data.

Only as an example, and without implying any limitation to such architecture, FIG. 1 depicts certain components that are usable in an example implementation of an embodiment. For example, servers and clients are only examples and do not to imply a limitation to a client-server architecture. As another example, an embodiment can be distributed across several data processing systems and a data network as shown, whereas another embodiment can be implemented on a single data processing system within the scope of the illustrative embodiments. Data processing systems (server 104, server 106, client 110, client 112, client 114) also represent example nodes in a cluster, partitions, and other configurations suitable for implementing an embodiment.

Intra-oral camera 122 includes one or more sensors, such as separate sensors, which detect defects in tooth using confined light injection and measure or scan tooth geometry and/or color by obtaining a plurality of images through projections and combining the projections to obtain a three-dimensional (3D) image. In an example, the intra-oral camera 122 captures data points as often as several thousand times each second, automatically detecting defects as well as registering the sizes and shapes of each tooth. It continuously sends this data to the connected computer's software.

Client application 120 or any other server application 116 implements an embodiment described herein. Client application 120 and/or server application 116 can use data from intra-oral camera 122 for contactless tooth defect detection and generate 3D jaw models of one or more teeth 124. Client application 120 can also execute in any of data processing systems (server 104 or server 106, client 110, client 112, client 114), such as client server application 116 in server 104 and need not execute in the same system as client 110.

On the other hand, client 110 may work "standalone", at least for a subset of applications, if not connected to network infrastructure 102 or in case of a failure in network infrastructure 102.

Machine learning engine 126 may identify a defect condition of the at least one tooth by classifying one or more surface light distribution images 128 based on computation of a homogeneity disturbance in the one or more surface light distribution images 128 caused by defective tooth material.

Machine learning engine 126 may be a part of, or separate from server 104 or clients 110, 112 and 114. The machine learning engine 126 may be trained based on a plurality of test surface light distribution images 128 that comprise healthy and defective tooth material data from each subgroup of teeth (anteriors, canines, premolars molars of upper and lower jaw) and various possible tooth defect locations and defect sizes.

Server 104, server 106, storage unit 108, client 110, client 112, client 114, may couple to network/communication infrastructure 102 using wired connections, wireless communication protocols, or other suitable data connectivity. Client 110, client 112 and client 114 may be, for example, personal computers or network computers.

In the depicted example, server 104 may provide data, such as boot files, operating system images, and applications to client 110, client 112, and client 114. Client 110, client 112 and client 114 may be clients to server 104 in this example. Client 110, client 112 and client 114 or some combination thereof, may include their own data, boot files, operating system images, and applications. Data processing environment 100 may include additional servers, clients, and other devices that are not shown. Server 104 includes the server application 116 that may be configured to implement one or more of the functions described herein for displaying restoration proposals in accordance with one or more embodiments.

Server 106 may include a search engine configured to search stored files such as images of patient teeth for comparison in response to a request for detecting tooth defects. In the depicted example, data processing environment 100 may be the Internet. Network/communication infrastructure 102 may represent a collection of networks and gateways that use the Transmission Control Protocol/Internet Protocol (TCP/IP) and other protocols to communicate with one another. At the heart of the Internet is a backbone of data communication links between major nodes or host computers, including thousands of dental practices, commercial, governmental, educational, and other computer systems that route data and messages. Of course, data processing environment 100 also may be implemented as a number of different types of networks, such as for example, an intranet, a local area network (LAN), or a wide area network (WAN). FIG. 1 is intended as an example, and not as an architectural limitation for the different illustrative embodiments.

Among other uses, data processing environment 100 may be used for implementing a client-server environment in which the illustrative embodiments may be implemented. A client-server environment enables software applications and data to be distributed across a network such that an application functions by using the interactivity between a client data processing system and a server data processing system. Data processing environment 100 may also employ a service-oriented architecture where interoperable software components distributed across a network may be packaged together as coherent business applications. Data processing environment 100 may also take the form of a cloud, and employ a cloud computing model of service delivery for enabling convenient, on-demand network access to a shared pool of configurable computing resources (e.g. networks, network bandwidth, servers, processing, memory, storage, applications, virtual machines, and services) that can be rapidly provisioned and released with minimal management effort or interaction with a provider of the service.

In case a server with a PMS 132 is available in the network and patient data as e.g. Xray images or entries in the patient files comprising tooth defect data or other data of concern as e.g. age of the patient, pulp vitality data of a tooth under investigation with the tooth defect detection device are accessible from different points in time, the machine learning engine 126 can include those data as part of a multimodal decision making approach.

For instance, a negative or weak pulp vitality, or hypersensitivity or suspect areas in a Xray of the tooth under investigation can be taken in consideration to increase the probability that an abnormal intensity distribution image has a higher probability of a severe tooth defect.

Same is valid, if diagnostic information of earlier points in time is available (earlier in time light intensity distribution images, x-rays, pulp vitality etc.). In such a case the actual light intensity distribution data can be put in context with diagnostic data from other modalities and information from other point in time and can be used to classify the actual situation and even predict from the time series of data the probability of further tooth defect development by the machine learning engine 126.

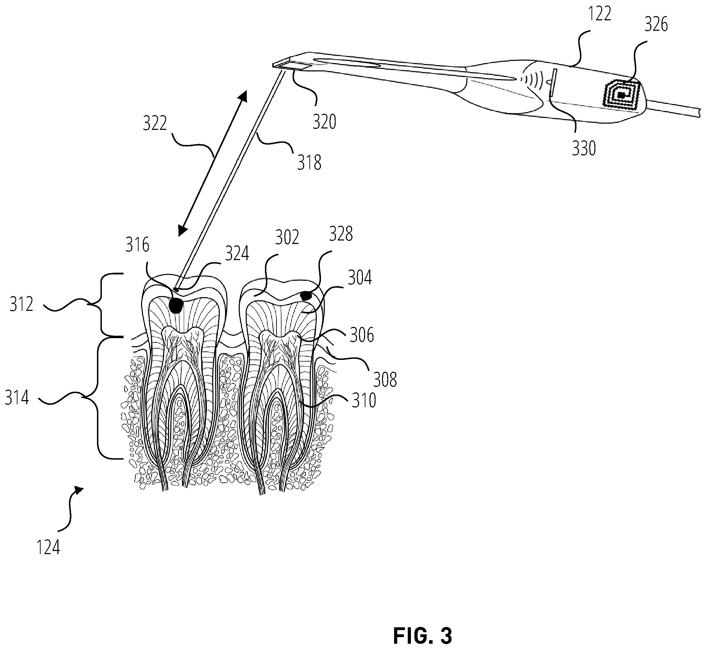

With reference to FIG. 2, this figure depicts a block diagram of a data processing system in which illustrative embodiments may be implemented. Data processing system 200 is an example of a computer, such client 110, client 112, client 114 or server 104, server 106, in FIG. 3, or another type of device in which computer usable program code or instructions implementing the processes may be located for the illustrative embodiments.

Data processing system 200 is described as a computer only as an example, without being limited thereto. Implementations in the form of other devices, may modify data processing system 200, such as by adding a touch interface, and even eliminate certain depicted components from data processing system 200 without departing from the general description of the operations and functions of data processing system 200 described herein.

In the depicted example, data processing system 200 employs a hub architecture including North Bridge and memory controller hub (NB/MCH) 202 and South Bridge and input/output (I/O) controller hub (SB/ICH) 204. Processing unit 206, memory 208, and graphics processor 210 are coupled to North Bridge and memory controller hub (NB/MCH) 202. Processing unit 206 may include one or more processors and may be implemented using one or more heterogeneous processor systems. Processing unit 206 may be a multi-core processor. Graphics processor 210 may be coupled to North Bridge and memory controller hub (NB/MCH) 202 through an accelerated graphics port (AGP) in certain implementations.

In the depicted example, local area network (LAN) adapter 212 is coupled to South Bridge and input/output (I/O) controller hub (SB/ICH) 204. Audio adapter 216, keyboard and mouse adapter 220, modem 222, read only memory (ROM) 224, universal serial bus (USB) and other ports 232, and PCI/PCIe devices 234 are coupled to South Bridge and input/output (I/O) controller hub (SB/ICH) 204 through bus 218. Hard disk drive (HDD) or solid-state drive (SSD) 226a and CD-ROM 230 are coupled to South Bridge and input/output (I/O) controller hub (SB/ICH) 204 through bus 228. PCI/PCIe devices 234 may include, for example, Ethernet adapters, add-in cards, and PC cards for notebook computers. PCI uses a card bus controller, while PCIe does not. Read only memory (ROM) 224 may be, for example, a flash binary input/output system (BIOS). Hard disk drive (HDD) or solid-state drive (SSD) 226a and CD-ROM 230 may use, for example, an integrated drive electronics (IDE), serial advanced technology attachment (SATA) interface, or variants such as external-SATA (eSATA) and micro- SATA (mSATA). A super I/O (SIO) device 236 may be coupled to South Bridge and input/output (I/O) controller hub (SB/ICH) 204 through bus 218.

Memories, such as main memory 208, read only memory (ROM) 224, or flash memory (not shown), are some examples of computer usable storage devices. Hard disk drive (HDD) or solid-state drive (SSD) 226a, CD-ROM 230, and other similarly usable devices are some examples of computer usable storage devices including a computer usable storage medium.

An operating system runs on processing unit 206. The operating system coordinates and provides control of various components within data processing system 200 in FIG. 2. The operating system may be a commercially available operating system for any type of computing platform, including but not limited to server systems, personal computers, and mobile devices. An object oriented or other type of programming system may operate in conjunction with the operating system and provide calls to the operating system from programs or applications executing on data processing system 200.

Instructions for the operating system, the object-oriented programming system, and applications or programs, such as server application 116 and client application 120 in FIG. 1, are located on storage devices, such as in the form of codes 226b on Hard disk drive (HDD) or solid-state drive (SSD) 226a, and may be loaded into at least one of one or more memories, such as main memory 208, for execution by processing unit 206. The processes of the illustrative embodiments may be performed by processing unit 206 using computer implemented instructions, which may be located in a memory, such as, for example, main memory 208, read only memory (ROM) 224, or in one or more peripheral devices.

Furthermore, in one case, code 226b may be downloaded over network 214a from remote system 214b, where similar code 214c is stored on a storage device 214d in another case, code 226b may be downloaded over network 214a to remote system 214b, where downloaded code 214c is stored on a storage device 214d.

A communications unit may include one or more devices used to transmit and receive data, such as a modem or a network adapter. A memory may be, for example, main memory 208 or a cache, such as the cache found in North Bridge and memory controller hub (NB/MCH) 202. A processing unit may include one or more processors or CPUs.

Where a computer or data processing system is described as a virtual machine, a virtual device, or a virtual component, the virtual machine, virtual device, or the virtual component operates in the manner of data processing system 200 using virtualized manifestation of some or all components depicted in data processing system 200. For example, in a virtual machine, virtual device, or virtual component, processing unit 206 is manifested as a virtualized instance of all or some number of hardware processing units 206 available in a host data processing system, main memory 208 is manifested as a virtualized instance of all or some portion of main memory 208 that may be available in the host data processing system, and Hard disk drive (HDD) or solid-state drive (SSD) 226a is manifested as a virtualized instance of all or some portion of Hard disk drive (HDD) or solid-state drive (SSD) 226a that may be available in the host data processing system. The host data processing system in such cases is represented by data processing system 200.

FIG. 3 shows a sketch illustrating a confined light injection into tooth material in accordance with an illustrative embodiment. The crown 312 of a tooth 124 comprises an outer portion which is enamel 302 and which protects an underlying dentin 304. The dentin 304 forms a major component of each tooth 124, is more elastic and softer than the enamel 302 and nourished by the pulp 306. The gum 308 or gingiva protects the jaw (alveolar) bone and roots of the teeth and covers the neck of each tooth. The tooth further includes blood vessels 310, and a root 314. To detect caries 316 and other tooth defects such as tooth cracks that may be present in the tooth 124, a spatially confined light 318 is projected at one or more illumination points 324 on the tooth 124. The one or more illumination points are clearly spatially separated. For example, the separation may be at least 3 times the diameter of an illumination spot. The spatially confined light 318, is injected by the confined light injector 320 of the intra-oral camera 122 into the tooth 124 in a first direction 322 and in a non-contact manner and backscattering of the light occurs to distribute the light within the tooth according to the scattering coefficients, absorption coefficients, refraction index and anisotropy factors of the tooth material or semitranslucent tooth restoration materials. The tooth is then recorded by an image sensor 326 sensitive to the wavelength of the light of the light injector. More than one wavelength can be used sequentially or simultaneous, depending on the type of sensor (monochromatic sensor or sensor with several color sensitive spectral channels) used for detection. This allows to differentiate between defects more on the surface of the tooth with visible or UV light or deeper under the surface in case of scattering in the NIR range. The image sensor 326 can be, for example, a complementary metal-oxide semiconductor (CMOS) sensor or a charge-coupled device (CCD) that may be used with wavelengths less than 1000nm and an indium gallium arsenide (InGaAs) sensor or a CQD sensor that may be used to cover wavelengths longer than 1000nm. The light may be returned in the same first direction 322 or in another direction and captured. In some cases, the spatially confined light 318 is focused on the dentin 304, especially through an illumination point 324 at an area of the enamel 302 that is thin. This disperses the light throughout the dentin 304 to produce a homogeneous background illumination. In the dentin 304, which is located underneath the enamel 302, the light is much more intensely scattered. Different brightness distributions are therefore, obtained within the tooth as a function of the illumination point 324. Only limited scattering of the light occurs in enamel due to the much lower scattering coefficient.

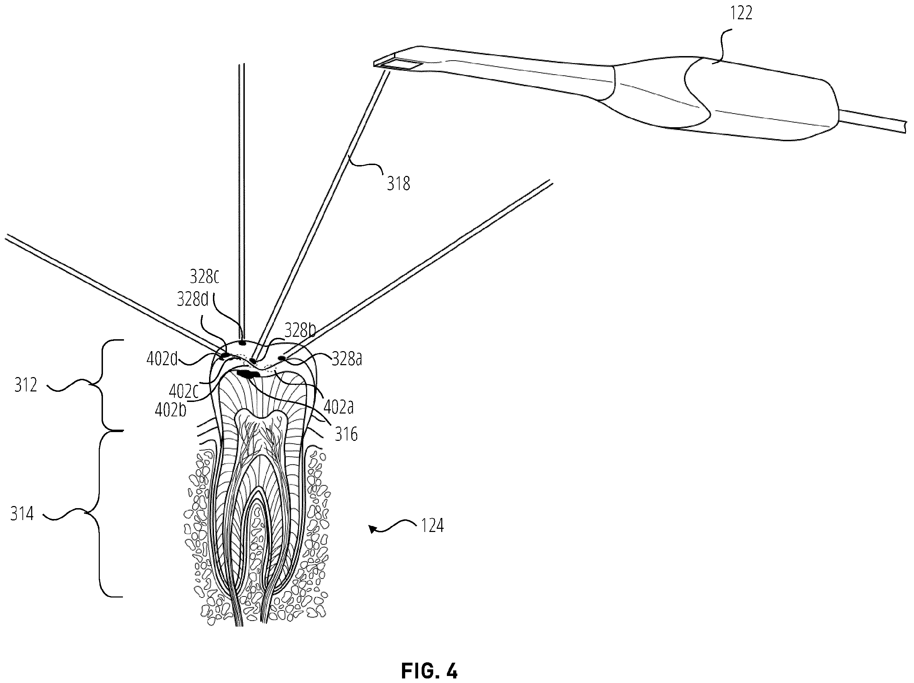

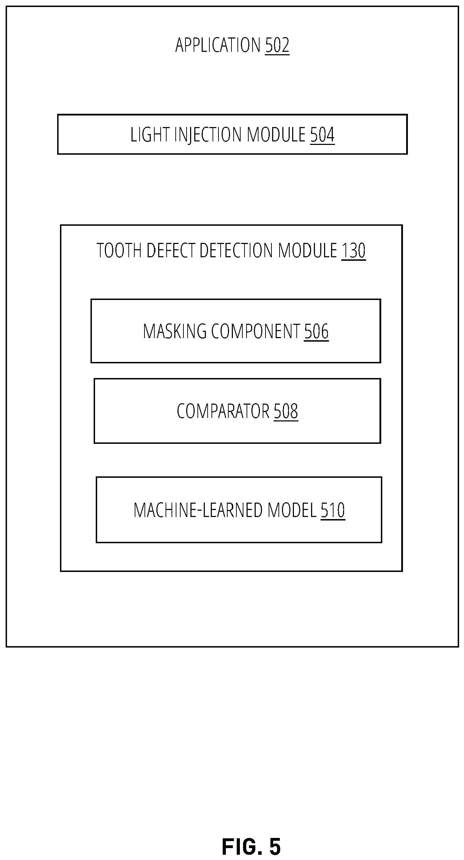

Tooth defects 328 such as caries 316 usually start in the tooth enamel in the area of the tooth crown and work their way in the direction of the dentin 304. The first optically detectable alterations in the tooth enamel 302 are produced in this way as zones with locally clearly increased scattering coefficients. If the caries 316 reach the dentin 304, a clear brownish discoloration occurs along the boundary between the enamel 302 and the dentin 304 due to an increased absorption coefficient, in particular-but not exclusively-in the blue and green spectral ranges. Caries starts generally on so-called predilection areas, among other things in fissures and below the contact point of two teeth. These are sometimes significantly difficult to diagnose visually since it eludes direct observation because the gingival papilla covers the interproximal space in the shape of a triangle and the adjacent teeth all stand closely together. However, it is possible to observe the tooth from the vestibular or lingual/buccal side. As seen in FIG. 4, the illumination points 324 of the spatially confined light 318 can be varied to achieve sufficient contrast with different observation directions. If the illumination point, with reference to the observation position, is positioned in front of the carious area with an increased scattering coefficient and / or increased absorption coefficient, this area is illuminated mainly on the observation side by light scattered partially in the enamel, and mainly scattered in the dentin. An area with a higher scattering coefficient is therefore visible to the observer (which is for example, an image scanner or sensor) as a brighter area, because the light is more highly scattered there than in healthy tooth enamel. Behind such an area of increased light absorption or scattering or after a refraction index mismatch, the illumination intensity originating from the point of light injection is reduced and therefore the area behind the defect of the tooth looks darker. A clear improvement in contrast is achieved if an image is stored with the light injection site in front of the caries and another with the light injection site behind the caries or tooth crack and these are subtracted one from each other. In a further aspect, a plurality of illumination points (for example, first illumination point 328a, second illumination point 328b, third illumination point 328c, and fourth illumination point 328d) may be sequentially illuminated with the spatially confined light 318. The spatially confined light 318 may be sequentially switched to illuminate the plurality of illumination points from the same intra-oral camera position, or the intra-oral camera 122 may be moved around to sequentially illuminate the plurality of illumination points. Due to the ability of the tooth defect absorb the confined light (such as what is seen with dentin caries), increase scattering of the confined light (such as what is seen with enamel demineralization), or cause loss of the confined light due to refraction index mismatch (such as what is seen with tooth cracks) shadows will be on the surface of the teeth. Spatially confined light (e.g., as one point or a grid of points) which is injected in the tooth is scattered to an extent by enamel, but to a much stronger extent in dentin, and therefore the light backscattered from the dentin of the tooth acts as a sort of "background illumination" coming out of the dentin part of the tooth. Part of this illumination may be absorbed by the caries causing a shadow to be formed on the surface of the tooth and the resulting light distribution on the tooth surface may be imaged by a sensor for analysis. Illuminating at the first illumination point 328a may generate a first shadow 402a on the surface of the tooth due to the caries 316 absorbing a portion of the diffuse illumination coming from inside the tooth; likewise, illuminating at a second illumination point 328b may generate a second shadow 402b; illuminating at a third illumination point 328c may generate a third shadow 402c; and illuminating at an illumination point 324 may generate a first shadow 402a. The positions of the shadows (shadows illustrated with dashed lines in FIG. 4) are shown for illustrative purposes and may in effect be at different locations based on the structure, position, and orientation of the spatially confined light 318. By aggregating the captured images corresponding to the different illumination points 324, a tooth defect detection module 130 (See FIG. 5 and FIG. 1) may compute information about the defective tooth material causing the shadow formation in the different images. In an example, the light distribution differences in the plurality of surface light distribution images can be detected and compared for calculating information about the defective tooth material. In another example, having a pre-knowledge about the light distribution in a tooth without tooth defects may enable the computation of information about newly formed defective tooth material in the same tooth at a future time by comparison of the light distribution differences relative to the pre-knowledge.

FIG. 5 is a block diagram illustrating an application 502 for contactless detection of defective tooth material. The application 502 may be a part of or may form the client application 120 or server application 116 of FIG. 1. The application 502 is illustrative and is not meant to be limiting as variations thereof may be obtained in view of the descriptions. The application 502 is configured to work with at least the components of the intra-oral camera 122 such as the confined light injector 320, and the image sensor(s) 326 to generate a spatially confined light 318, projecting the spatially confined light in a first direction via the confined light injector 320, at an illumination point of the at least one tooth, recording, by an image sensor, one or more surface light distribution images 128, and compute, by a tooth defect detection module, a defect condition of the at least one tooth based on classifying the one or more surface light distribution images 128 according to the changes in light distribution in the one or more surface light distribution images 128 caused by defective tooth material in comparison to a light distribution as would be expected in teeth without defect.

The application 502 comprises a light injection module 504, and a tooth defect detection module 130 which further comprises a comparator 508, a machine-learned model 510, and a masking component 506.

The light injection module 504 and the confined light injector 320 may work together or separately to provide specifications for generation and spatial confinement of the spatially confined light 318 and/or instructions for projecting the spatially confined light 318 to an illumination point 324. Thus, processes performed by the light injection module 504 may also be fully or partially achievable by the confined light injector 320. In an aspect, the light injection module 504 provides one or more spatially confined light spots using, for example, one or more laser beam(s) for light injection. Further the light injection module 504 may sequentially switch the spatially confined light. The light injection module 504 may further focus the spatially confined light 318 slightly subsurface such that it is focused on the dentin 304. In case of using NIR light, a visual aiming beam may also be provided to provide a visualization of the illumination point 324 during scanning. A sequential switching of the visual aiming beam may be configured to be distinguishable and separate from the spatially confined light 318. Further, the light injection module 504 controls a spatial filter to block out the light of the direct illumination spot from the image sensor 326. In another aspect, the light injection module 504 may inject the spatially confined light 318 at an illumination point 324 that is not in the viewing field of the intra-oral camera 122 or sensor thereby alleviating a need for masking out the area of direct illumination. In general, the light injection module 504 adapts various techniques for ambient light reduction such as laser beam modulation, background subtraction, and use of special image sensors comprising near- infrared (NIR) sensitive pixels in addition to RGB sensors (Mosaic Sensors) for spectral resolution of the spatial light differences caused by a tooth defect in combination with multiple light injection wavelength.

Further, the light injection module 504 configures the intra-oral camera 122 to collect images sequentially with different positions of the illumination points 324 by sequentially switching more than one laser beam. Due to the unchanged relative position between tooth and intra-oral camera 122, due to capture of several images within a short time period such as a few 10ms, it may be relatively easier to combine images collected sequentially with different illumination point 324. Specifically, laser beams may be used to sequentially provide more than one light injection point. This allows a more accurate determination of a tooth defect when, for example, 4 images with 4 different light injection points are combined from the same viewing direction in four consecutive frames. In an aspect, the light injection module 504 configures the projection of not only spatially confined light 318 but also of other non-spatially confined light sources such as LED light onto the tooth 124. The reflected light is then focused by a lens onto an image sensor that is sensitive to the non-spatially confined light. The application 502 overlays the one or more surface light distribution images 128 corresponding to the spatially confined light with visible light information and stores the overlay as a video stream. The visible light information may be produced with light of wavelength between, for example, 400nm - 750nm, including 450 nm which can be used for 3D acquisition. In other embodiments, the wavelength may range from 850 - 1600 nm, or from 850 - 1000 nm. In an aspect, the light injection module 504 configures the projection of the spatially confined light 318 to comprise predetermined dimensions such that a diameter of the illumination point 324 is from 50µm to 1mm or from 100µm to 500 µm, or from 0.5mm to 0.75mm. Further, polarized light may be used to reduce direct surface reflections.

In an aspect, the tooth defect detection module 130 uses a plurality of surface light distribution images/frames from different observing positions to compute a convergence to a mutual tooth defect position, size and/or type, and thus a likelihood that a tooth defect is present. Further, the tooth defect detection module 130 uses information about the 3D data of the scanning process to determine the relative position between the intra-oral camera 122 and the tooth. By combining a plurality of surface light distribution images captured sequentially using different positions of the illumination points 324 with the knowledge of the relative position of the intra-oral camera 122 and the tooth, the tooth defect detection module 130 can compute tooth defect information by arranging the images taken from different positions into the same coordinate reference frame by providing a coordinate transformation. This enables to scale and properly overlay the images taken from different relative positions for comparison/further computations.

In another aspect, the detection is aided by correlation of the surface light distribution images 128 with known surface light distribution images that are representative of tooth defects. This may further be aided by the use of the masking component 506, the comparator 508 and/or the machine-learned model 510. Responsive to strong back reflection occurring at the illumination point 324 being masked out by the masking component 506, an evaluable transillumination volume scattering image may be obtained in the remaining areas of the surface light distribution images 128, the content of which can be used to draw conclusions about structural damage to the tooth structure. Specifically, a plurality of images from different directions may be combined to algorithmically eliminate those areas which are not usable due to the surface reflections at the illumination point by the masking component 506. The illumination point may be computed from the knowledge of the position of the intra-oral camera in relation to the tooth or can be determined by selecting pixels with exposure values that exceed a defined high threshold to define the area to be masked around it. The masked-out areas of individual surface light distribution images 128 can be filled or reconstructed using other surface light distribution images 128 from other viewing positions in which those corresponding areas of the tooth have not been masked out.

The tooth defect detection module 130 may in some cases use of images taken at different points in time to calculate a progression of caries 316 or tooth cracks. Fillings may also be automatically detected and stored in a practice management system. Using information from previous examinations and information stored in the practice management system, pre-knowledge about patient specific tooth situations may be used in the contactless tooth defect detection.

Further, a plurality of the surface light distribution images 128 from different camera positions can be combined to form one "overall" image. In an aspect, light may be injected into adjacent or neighboring teeth for interproximal caries detection to provide a more indirect illumination of the tooth defect. Further, in a multimodal approach the tooth defect detection module 130 may combine NIR images or surface light distribution images 128, created with the spatially confined light injection, with a full field VIS illumination to, for example, differentiate caries from stains. Stains are comparatively less visible and sometimes invisible in NIR illumination but may be visible in visible light. Therefore, using both wavelength ranges allow the differentiation between stains present on teeth e.g., from smoking, coffee, and tea. Surface light distribution images 128 may also be combined with fluorescence imaging in a multimodal approach. The surface light distribution images 128 may further be shown as live images during scanning.

The tooth defect detection module 130 may use the comparator 508 to compare the surface light distribution images 128 to images in a database for contactless tooth defect detection purposes. To this effect, a simple view of the light distribution in each frame may be provided. An operator moves the intra-oral camera 122 over areas of interest and observes the surface light distribution images 128, potentially overlaid with visible light information as a "Video Stream". A plurality of surface light distribution images 128 from different scanner positions are combined to form one "overall" image as is done with the 3D scanning data. To optimize the outcome, an amount of "pre-knowledge" may be used. Firstly, typical tooth defect positions such as occlusal pits, fissures and the interproximal position slightly below contact points can be assumed to occur with a higher probability. The patient specific position of fillings and ideally knowledge of the material of the fillings from previous examinations may also be retrieved from a database of the PMS 132. A database may also be formed to provide the light distribution of typical healthy teeth and as well as the light distribution of unhealthy teeth. The light distributions may be formed for different types of tooth defects such as caries or cracks, on different tooth positions, for different tooth types (incisor, canine, pre-molar, molar) and on different jaws (e.g., the upper or lower jaw). The data base can be created with real cases from extracted or in-situ teeth and/or Monte-Carlo simulation of light propagation in virtual tooth models. In the case of extracted teeth or in-situ teeth, teeth with different defects are imaged with the light injection method and the images are stored. Afterwards extracted teeth are either imaged with µCT or sliced and the defect size and position are attached to the respective image as information, while from in-situ teeth x-ray images and visual assessment can be used as reference. In case of Monte Carlo simulation, µCT data of extracted teeth or synthetic tooth compositions comprising enamel and dentin layers and tooth defects of any size and position can be used as geometry information and the light injected at a certain tooth position can be traced by the Monte Carlo simulation. As result an image from each desired observation position is available based on the light propagation in simulated tooth structure and can be stored in the database.

The tooth defect detection module 130 then compares, using the comparator 508, the surface light distribution images with the database, taking into consideration the patient specific historical data to make a determination of the fit of the surface light distribution images 128 to the existing images of the database. In one aspect, responsive to a plurality of frames from different observing positions agreeing on a convergence to a mutual caries position, a size and/or a type, the tooth defect detection module makes a determination of at least the presence of the tooth defect 328. Other information about the tooth defect 328 may also be computed, such as the size, position, type, and shape. In an aspect, simulation images with the best fit can be combined to form an "overall" image that represents the most likely tooth situation. In an aspect, to detect caries on filling margins, knowledge of the shape and material (semi-translucent, optically dense, etc.) known from the 3D scans, visual images, x-rays, etc., can be used in by the comparator 508 to determine the most likely caries situation around a filling.

In another aspect, a machine-learned model 510 is used for AI based classification of surface light distribution images 128. The machine-learned model 510 may be, for example, an image transformer networks or convolutional neural network.

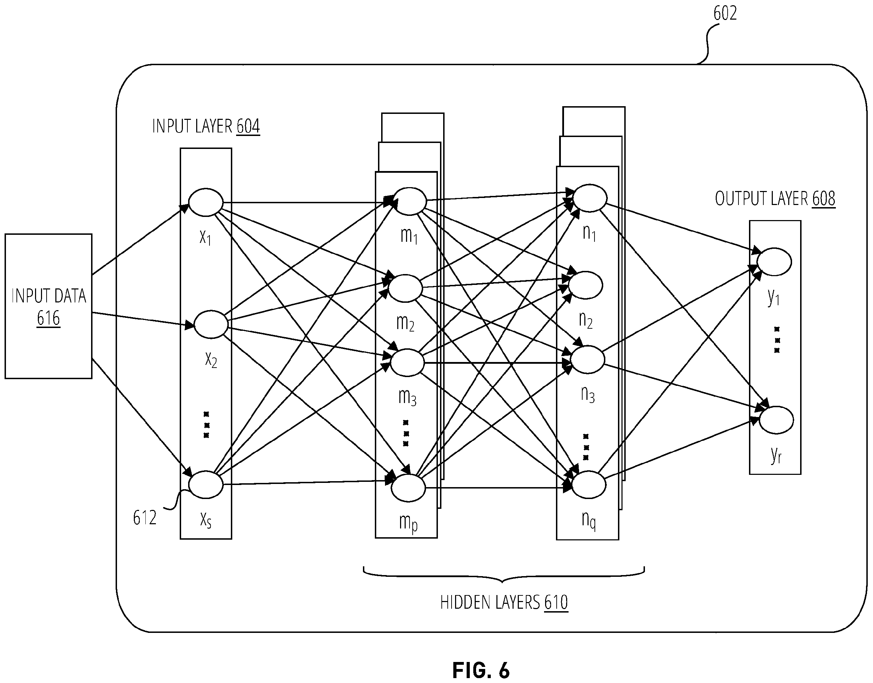

FIG. 6 shows a block diagram illustrating a structure of a neural network 602 according to an embodiment of the present invention in which the machine-learned model 510 is a neural network 602. The neural network 602 comprises a plurality of layers including an input layer 604, one or more hidden layers 610 and an output layer 608. Each layer may comprise of one or more nodes 612, indicated by circles. Information may flow from the input layer 604 to the output layer 608.

The node 612 has an input and an output and the nodes of the input layer 604 are passive, meaning they the data thereof is unmodified. For example, the nodes 612 of the input layer 604 may each receive an input data 616 (e.g., a surface light distribution image 128) on their input and copy the value to their multiple outputs. Conversely, the nodes of the hidden layers 610 and output layer 608 may be active, therefore being able to modify the data. In an example structure, each value from the input layer 604 is copied and sent to all of the hidden nodes. The values entering the hidden nodes are multiplied by weights, which are a set of predetermined numbers associated with each of the hidden nodes. The weighted inputs are then be summed to produce a single number.

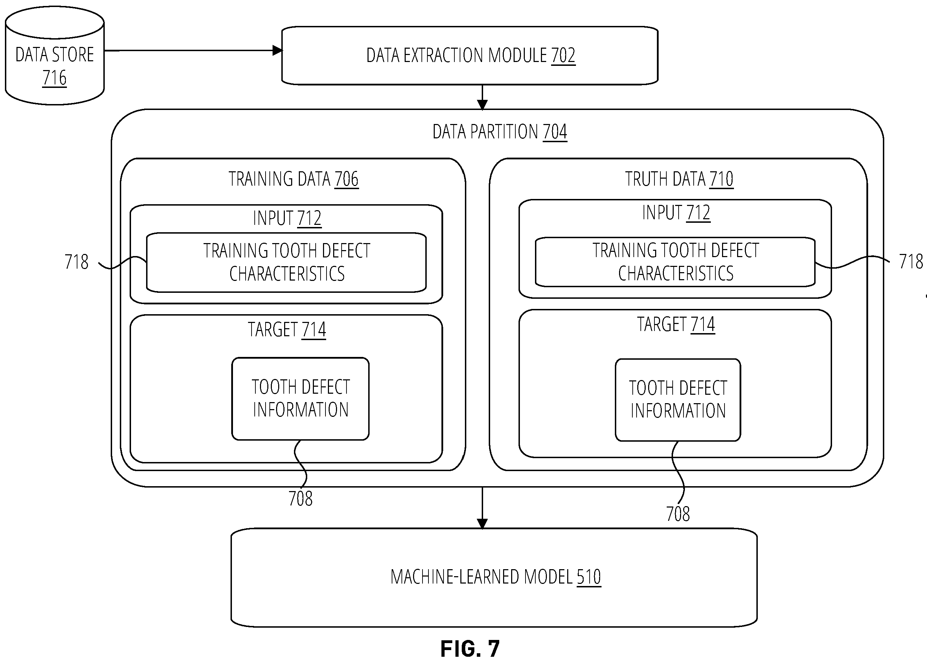

In an embodiment, the neural network 602 may use as input at tooth defect characteristic information such as least one of, one or more surface light distribution images 128, 3D data of the intra-oral camera, and visible light information, patient specific tooth information. Thus, the number of nodes 612 in the input layer 604 is proportional to the dimensions of the input data. The machine-learned model 510 is further described with respect to training architecture of FIG. 7.

FIG. 7 shows a block diagram illustrating a training of the machine-learned model 510 for contactless tooth defect detection. The training is performed by extracting, via the data extraction module 702, a dataset of input data from the data store 716. A portion of the data may be generated using a known dataset of surface light distribution image 128 that are known to include tooth defects 328 and that can serve as truth data for training the machine-learned model 510. Further, a dataset generated using the intra-oral camera on various teeth that include tooth defects 328 and on various teeth that include no tooth defects and under various conditions such as different tooth positions, tooth types, jaws, patients, etc. may be used as training data. The training data 706 and truth data 710 are labelled as input 712 and targets 714 for training. One or more of the training tooth defect characteristics 718 may be used as at least part of the input 712 (training or validation inputs) and one or more of the tooth defect information 708 (such as the presence of a tooth defect, size of a tooth defect etc.) is used as a target 714 (training or validation targets). In an aspect, the input 712 can in some cases be preprocessed to reduce dimensionality thereof. Training data 706 is used to train the model by giving the model an input and measuring the target, while truth data 710 is used to tune the hyperparameters of the machine-learned model 510 and make decisions about the model's structure, such as selecting between different architectures. Even further, another set of data, test data, can be used to evaluate the final performance of the machine-learned model 510 and to estimate its classification ability to new, unseen surface light distribution images 128.

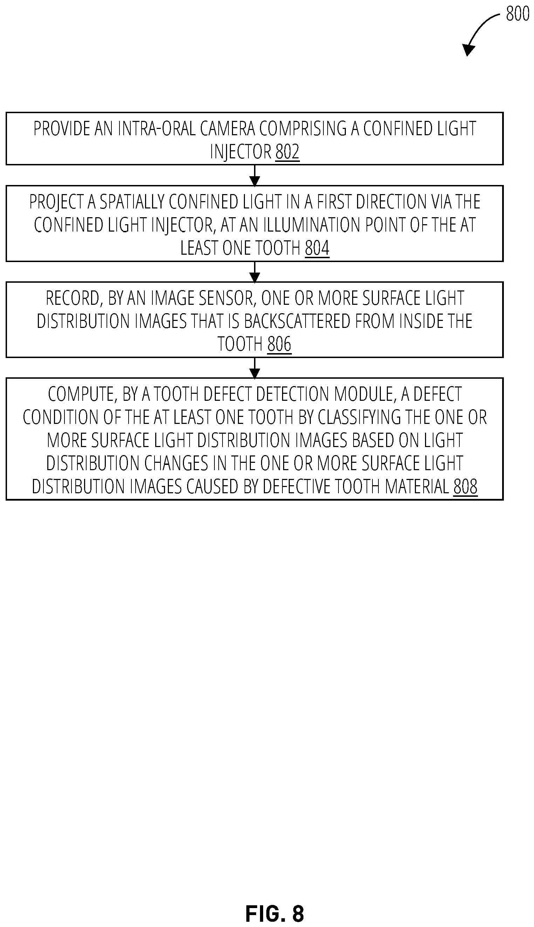

FIG. 8 depicts a contactless tooth defect detection routine 800 in accordance with an illustrative embodiment. The contactless tooth defect detection routine 800 may be performed by the tooth defect detection module 130. In block 802, an intra-oral camera comprising a confined light injector is provided. In block 804, tooth defect detection module 130 projects a spatially confined light in a first direction via the confined light injector, at an illumination point of the at least one tooth. In block 806, tooth defect detection module 130 records, by an image sensor, one or more surface light distribution images obtained by capturing light that is backscattered from inside the tooth. The captured light may be captured from the same direction as the first direction or from a different direction. In block 808, tooth defect detection module 130 computes a defect condition of the at least one tooth by classifying the one or more surface light distribution images-based computation of light distribution changes in the one or more surface light distribution images caused by defective tooth material.

Thus, a computer implemented method, system or apparatus, and computer program product are provided in the illustrative embodiments for contactless tooth defect detection and other related features, functions, or operations. Where an embodiment or a portion thereof is described with respect to a type of device, the computer implemented method, system or apparatus, the computer program product, or a portion thereof, are adapted or configured for use with a suitable and comparable manifestation of that type of device.

Where an embodiment is described as implemented in an application, the delivery of the application in a Software as a Service (SaaS) model is contemplated within the scope of the illustrative embodiments. In a SaaS model, the capability of the application implementing an embodiment is provided to a user by executing the application in a cloud infrastructure. The user can access the application using a variety of client devices through a thin client interface such as a web browser, or other light-weight client-applications. The user does not manage or control the underlying cloud infrastructure including the network, servers, operating systems, or the storage of the cloud infrastructure. In some cases, the user may not even manage or control the capabilities of the SaaS application. In some other cases, the SaaS implementation of the application may permit a possible exception of limited user-specific application configuration settings.

The present invention may be a system, a method, and/or a computer program product at any possible technical detail level of integration. The computer program product may include a computer readable storage medium (or media) having computer readable program instructions thereon for causing a processor to carry out aspects of the present invention.

The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, including but not limited to computer-readable storage devices as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present invention may be assembler instructions, instruction-set-architecture (ISA) instructions, machine instructions, machine dependent instructions, microcode, firmware instructions, state-setting data, configuration data for integrated circuitry, or either source code or object code written in any combination of one or more programming languages, including an object oriented programming language such as Smalltalk, C++, or the like, and procedural programming languages, such as the "C" programming language or similar programming languages. The computer readable program instructions may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider). In some embodiments, electronic circuitry including, for example, programmable logic circuitry, field-programmable gate arrays (FPGA), or programmable logic arrays (PLA) may execute the computer readable program instructions by utilizing state information of the computer readable program instructions to personalize the electronic circuitry, in order to perform aspects of the present invention.

Aspects of the present invention are described herein with reference to flowchart illustrations and/or block diagrams of methods, apparatus (systems), and computer program products according to embodiments of the invention. It will be understood that each block of the flowchart illustrations and/or block diagrams, and combinations of blocks in the flowchart illustrations and/or block diagrams, can be implemented by computer readable program instructions.

These computer readable program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions may also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram block or blocks.

The computer readable program instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the blocks may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

### Conclusion

Although techniques using contactless tooth defect detection, and apparatuses including, tooth defect detection module have been described in language specific to features and/or methods, it is to be understood that the subject of the appended claims is not necessarily limited to the specific features or methods described. Rather, the specific features and methods are disclosed as example implementations of contactless tooth defect detection.

Example 1: A method comprising: providing an intra-oral camera comprising a confined light injector; projecting a spatially confined light in a first direction via the confined light injector, at an illumination point of the at least one tooth; recording, by an image sensor, one or more surface light distribution images received via captured light that is backscattered from the tooth; computing, by a tooth defect detection module, a defect condition of the at least one tooth by classifying the one or more surface light distribution images based on computation of a change in light distribution in the one or more surface light distribution images caused by a defective tooth material, wherein the spatially confined light is projected at the illumination point in a contactless manner.

Example 2: The method of example 1, wherein the defect condition is defined by caries, demineralization, or cracks in the at least one tooth.

Example 3: The method of example 1 or 2, further comprising: scanning an entire jaw by projecting the spatially confined light at a plurality of illumination points on a plurality of teeth, either alone for the purpose of tooth defect detection or in combination with the scanning of the 3D geometry of the teeth as needed to create a 3D data set of the tooth.

Example 4: The method of any previous example, projecting the spatially confined light in a plurality of direction, by the confined light injector, at a plurality of illuminations of the at least one tooth; recording a plurality of surface light distribution images from different directions; computing the defect condition based on at least two surface light distribution images, from change in light distribution caused by the defective tooth material; wherein the computing of the defect condition converges to a mutual tooth defect condition for the at least two surface light distribution images.

Example 5: The method of any previous example, wherein the mutual tooth defect condition comprises at least one of a presence of the tooth defect, a position of the tooth defect, and a geometry of the tooth defect.

Example 6: The method of example 4, further comprising: masking out back reflection from the one or more surface light distribution images to obtain one or more corresponding evaluable transillumination volume scattering image data for remaining areas of the one or more surface light distribution images, wherein the back reflection is caused by the direct surface reflection and close-to-surface volume scattering at the illumination point, of at least one tooth.

Example 7: The method of example 6, further comprising: for at least one surface light distribution image of the one or more surface light distribution images, reconstructing tooth information masked out from said masking using at least one other surface light distribution image.

Example 8: The method of example 4, further comprising: combining a plurality of the one or more surface light distribution images to form an overall image.

Example 9: The method of any previous example, further comprising: overlaying the one or more surface light distribution images with visible light information as a live or stored video stream.

Example 10: The method of example 1, wherein the illumination point is chosen to be at a point that is not in an image field of the sensor, thereby reducing or eliminating a masking out process of back reflection.

Example 11: The method of any previous example, wherein the spatially confined light is a laser beam in a near-infrared (NIR) wavelength range.

Example 12: The method of any previous example, wherein the spatially confined light is polarized to suppress back reflection in combination with a cross polarized filter at the image sensor.

Example 13: The method of any previous example, wherein the spatially confined light causes a diffuse illumination from inside of the at least one tooth with a highest intensity at the illumination point and decreasing intensity into a periphery of the illumination point.

Example 14: The method of any previous example, wherein the image sensor is sensitive to a wavelength range of the spatially confined light.

Example 15: The method of any previous example, further comprising: computing a location of the illumination point based on a position of the intra-oral camera relative to the at least one tooth using the 3D geometry information captured by the intra-oral scanner to generate a 3D data set of said at least one tooth.

Example 16: The method of any previous example, further comprising: computing a location of the illumination point based on pixels of the one or more surface light distribution images with exposure values that exceed a threshold.

Example 17: The method of any previous example, wherein the computing is performed by a machine-learned model that is trained based on at least a plurality of test surface light distribution images that include defective and healthy tooth material data.

Example 18: The method of examples 1-17, wherein the computing is performed by comparison of the one or more surface light distribution images with a database of stored surface light distribution images that include defective and healthy tooth material data.

Example 19: The method of example 18, wherein the database is generated based on light propagation in extracted or in-situ actual teeth and/or Monte-Carlo simulation of light propagation in virtual tooth models.

Example 20: The method of any previous example, further comprising: projecting the spatially confined light in the first direction into a neighboring tooth to provide an indirect illumination of an interproximal caries or crack.

Example 21: A system comprising: an intra-oral camera including a confined light injector, a processor, and a memory storing instructions that, when executed by the processor, configure the system to perform any of the examples 1 - 20.

Example 22: A non-transitory computer readable storage medium storing one or more programs that when executed by a processor cause the intra-oral camera to perform any of the examples 1 - 20.

## Claims

1. A method comprising:
providing an intra-oral camera comprising a confined light injector;
projecting a spatially confined light in a first direction via the confined light injector, at an illumination point of the at least one tooth;
recording, by an image sensor, one or more surface light distribution images received via captured light backscattered from the tooth;
computing, by a tooth defect detection module, a defect condition of the at least one tooth by classifying the one or more surface light distribution images based on computation of a change in light distribution in the one or more surface light distribution images caused by a defective tooth material,
wherein the spatially confined light is projected at the illumination point in a contactless manner.

2. The method of claim 1, wherein the defect condition is defined by caries, demineralization, and/or cracks in the at least one tooth.

3. The method of claim 1, further comprising:
scanning an entire jaw by projecting the spatially confined light at a plurality of illumination points on a plurality of teeth.

4. The method of claim 1, projecting the spatially confined light in a plurality of directions, by the confined light injector, at a plurality of illumination points of the at least one tooth;
recording a plurality of surface light distribution images from different directions;
computing the defect condition based on at least two surface light distribution images from the change in light distribution caused by the defective tooth material;
wherein the computing the defect condition converges to a mutual tooth defect condition for the at least two surface light distribution images.

5. The method of claim 4, wherein the mutual tooth defect condition comprises at least one of a presence of the tooth defect, a position of the tooth defect, and a geometry of the tooth defect.

6. The method of claim 4, further comprising:
masking out back reflection from the one or more surface light distribution images to obtain one or more corresponding evaluable volume scattering image data for remaining areas of the one or more surface light distribution images,
wherein the back reflection is caused by a surface reflection or close-to-surface volume scattering at the illumination point.

7. The method of claim 6, further comprising:
for at least one surface light distribution image of the one or more surface light distribution images, reconstructing tooth information masked out from said masking using at least one other surface light distribution image.

8. The method of claim 4, further comprising:
combining a plurality of the one or more surface light distribution images to form an overall image.

9. The method of claim 1, further comprising:
overlaying the one or more surface light distribution images with visible light information as a live or stored video stream.

10. The method of claim 1, wherein the illumination point is chosen to be at a point that is not in an image field of the sensor, thereby reducing or eliminating a masking out process of back reflection.

11. The method of claim 1, wherein the spatially confined light is a laser beam in a near-infrared (NIR) wavelength range.

12. The method of claim 1, wherein the spatially confined light is polarized and a cross-polarized filter is disposed in front of the image sensor to suppress direct back reflection from the tooth surface.

13. The method of claim 11, wherein the spatially confined light causes a diffuse illumination from inside of the at least one tooth with a highest intensity at the illumination point and decreasing intensity into a periphery of the illumination point.

14. The method of claim 1, wherein the image sensor is sensitive to a wavelength range of the spatially confined light.

15. The method of claim 1, further comprising:
computing a location of the illumination point based on a position of the intra-oral camera relative to the at least one tooth using 3D geometry information captured by the intra-oral camera to generate a 3D data set of the at least one tooth.

16. The method of claim 1, further comprising:
computing a location of the illumination point based on pixels of the one or more surface light distribution images with exposure values that exceed a threshold.

17. The method of claim 1, wherein the computing is performed by comparison of the one or more surface light distribution images with a database of stored surface light distribution images that include defective and healthy tooth material data.

18. The method of claim 1, wherein the computing is performed by a machine-learned model that is trained based on at least a plurality of test surface light distribution images that include defective and healthy tooth material data.

19. The method of claim 17, wherein the database is generated based on light propagation in extracted or in-situ teeth and/or Monte-Carlo simulation of light propagation in virtual tooth models.

20. The method of claim 1, further comprising:
projecting the spatially confined light in the first direction into a neighboring tooth to provide an indirect illumination of an interproximal caries or crack.

21. A system comprising:
an intra-oral camera including a confined light injector, a processor, and a memory storing instructions that, when executed by the processor, configure the system to:
project a spatially confined light in a first direction via the confined light injector, at an illumination point of the at least one tooth;
record, by an image sensor, one or more surface light distribution images received via captured light backscattered from the tooth; and
compute, by a tooth defect detection module, a defect condition of the at least one tooth by classifying the one or more surface light distribution images based on computation of a change in light distribution in the one or more surface light distribution images caused by defective tooth material,
wherein the spatially confined light is projected at the illumination point in a contactless manner.

22. A non-transitory computer readable storage medium storing one or more programs that when executed by a processor cause the intra-oral camera to:
project a spatially confined light in a first direction, at an illumination point of the at least one tooth;
record one or more surface light distribution images received from captured light backscattered from the tooth; and
compute a tooth defect condition of the at least one tooth by classifying the one or more surface light distribution images based on computation of change in light distribution in the one or more surface light distribution images caused by defective tooth material,
wherein the spatially confined light is projected at the illumination point in a contactless manner.
